# EUROPÄISCHE PATENTSCHRIFT

(11) **EP 2 422 839 B1**
(45) Veröffentlichungstag und Bekanntmachung des Hinweises auf die Patenterteilung: **03.04.2013**
(21) Anmeldenummer: 11176444.5
(22) Anmeldetag: 03.08.2011
(51) Int. Cl.: A61N 1/05, H01R 4/20, A61N 1/02

(54) **Elektrodenkatheter, insbesondere zur Kardialtherapie**
Electrode catheter, in particular for cardiac therapy
Cathéter à électrodes, notamment pour la thérapie cardiaque

(30) Priorität: 31.08.2010 US 378406 P
(43) Veröffentlichungstag der Anmeldung: 29.02.2012
(73) Patentinhaber: BIOTRONIK SE & Co. KG, 12359 Berlin (DE)
(72) Erfinder: Weitzig, Pierre, 10249 Berlin (DE); Palm, Jochen, 15831 Mahlow (DE); Jadwizak, Detmar, 15537 Erkner (DE); Fründt, Carsten, 13057 Berlin (DE)
(74) Vertreter: Lindner-Vogt, Karin L.

(56) Entgegenhaltungen:
- US-A- 4 592 372
- US-A- 5 330 522
- US-A1- 2002 055 764
- US-A1- 2004 064 174
- US-A1- 2005 004 642
- US-A1- 2006 037 195

## Beschreibung

Die Erfindung betrifft einen Elektrodenkatheter insbesondere zur Kardialtherapie mit den im Oberbegriff des Patentanspruches 1 angegebenen Merkmalen.

Derartige Elektrodenkatheter sind in unterschiedlichsten Ausführungsformen durch offenkundige Vorbenutzung auch der vorliegenden Anmelderin bekannt und umfassen als Basiskomponenten einen langgestreckten, schlauchartigen Katheterkörper, mindestens eine Elektrode zur Abgabe oder Messung eines elektrischen, insbesondere elektrokardialen Signals über ihre äußere Elektrodenkontaktfläche, mindestens eine erste Zuleitung zum elektrischen Anschluss dieser Elektrode und mindestens eine zweite Zuleitung zum elektrischen Anschluss einer weiteren Elektrode. Typischerweise sind diese Elektroden als zwei am distalen Ende des Elektrodenkatheters mit axialem Abstand sitzende Ringelektroden oder eine Ring- und eine Kopfelektrode ausgelegt, über die Reizleitungssignale des Herzens gemessen oder elektrische Impulse z.B. zum Beenden eines Vorhofflimmerns abgegeben werden.

Für die Konfiguration der Elektrodenzuleitungen sind grundsätzlich drei unterschiedliche Typen bekannt. Der erste Typ ist die so genannte koradiale Wendel, bei der die zwei Zuleitungen analog einem Gewinde mit zwei oder mehr Gängen parallel zueinander gewickelt und voneinander isoliert sind. Bei solchen koradialen Wendeln werden die Drähte zur Kontaktierung einer Ringelektrode aus dem Wendelverbund tangential ausgelenkt und abisoliert. Die Drähte werden dann zwischen einer Innen- und Außenhülse geklemmt und gecrimpt oder verschweißt.

Nachteilig beim Anschließen und Durchführen der Zuleitungen bei solchen koradialen Wendeln ist die Tatsache, dass das tangentiale Herausführen der Drähte und das Einklemmen zwischen zwei Hülsen vergleichsweise viel Bauraum erfordert, was das Ziel eines möglichst kleinen, isodiametrischen Elektrodendurchmessers erschwert. Ferner ist die Wickeltechnik sehr aufwendig und damit kostenintensiv.

Der zweite Typ von Elektrodenzuleitungen ist die koaxiale Wendel welche über einen wendelförmigen Innenleiter, der beispielsweise zu einer Kopfelektrode führt, und einen wendelförmigen Außenleiter, der eine proximal davor angeordnete Ringelektrode kontaktiert verfügt. Die Wendeln werden mit der Kopf- bzw. Ringelektrode mittels Schweißen oder Crimpen verbunden. Nachteilig bei diesem Zuleitungstyp ist die Tatsache, dass aufgrund der beiden koaxial ineinander sitzenden Wendeln das Erreichen eines möglichst kleinen Elektrodendurchmessers im mm-Bereich problematisch ist. Auch für multipolare Elektrodentypen kann diese Zuleitungstechnik praktisch ausgeschlossen werden.

Schließlich ist als Zuleitungstechnik noch ein so genannter Mehrlumenaufbau bekannt, bei dem ein schlauchartiger Katheterkörper entlang des Elektrodenkatheters über mindestens zwei Lumen verfügt. Durch eines der beiden Lumen verläuft ein dünnes Drahtseil zur Ringelektrode, durch das andere Lumen wird eine Wendel beispielsweise zu einer Kopfelektrode geführt. Das Drahtseil verläuft dabei axial ausgerichtet zur Wendel und wird mit der Ringelektrode verschweißt oder gecrimpt. Die Wendel führt bis zum Elektrodenkopf und wird mit dieser ebenfalls verschweißt oder gecrimpt.

Nachteilig bei diesem Mehrlumenaufbau ist die Tatsache, dass aufgrund der Einhaltung von Mindestwandstärken für die Außenisolation die Auslegung einer multipolaren Elektrode schwierig ist. Der Elektrodenaufbau ist ferner unsymmetrisch und es existiert, da die Ringelektrode nur über ein Seil mit dem Anschlussstecker verbunden ist, keine Redundanz für die elektrische Kontaktierung der Ringelektrode. Auch kann die Dauerfestigkeit der Kontakte zwischen dem Zuleitungs-Drahtseil und der jeweiligen Elektrode problematisch sein.

Die US 6,249,708 B 1 offenbart einen Mehrleiter-Elektrodenkatheter, bei dem eine zentrale Wendel bis zu einer Kopfelektrode geführt und mit dieser verschweißt ist. Um diese Wendel ist ein Isolierkörper versehen, der in seiner Umfangsfläche mit verdrillt laufenden Rillen versehen ist. In diesen sind jeweils Zuleitungen für eine Ringelektrode eingesetzt. Auch diese Konstruktion ist den Koaxial-Wendeln mit den oben beschriebenen Nachteilen zuzuordnen.

Die US 6,757,970 B1 zeigt einen Mehrfachelektrodenkatheter, bei dem die Elektrodenzuleitungen gemeinsam in einer mehrgängigen Wendel geführt und die Enden aus dem Wendelverbund radial weggeführt sind. Die Kontaktierung der abstehenden Enden erfolgt in aufwendiger Weise über das Umbiegen von Kontaktlaschen an den streifenförmigen Ringelektroden, wobei durch das Umbiegen die abstehenden Enden der Zuleitungen eingeklemmt werden. Anschließend muss die Streifenkonfiguration der Kontakte noch durch ein Werkzeug zum Einrollen in die lang gestreckt zylindrische Form des Katheters gezogen werden. Diese Konstruktion mit ihrer spezifischen Fertigungsweise erscheint im Hinblick auf die filigrane Ausgestaltung von Elektrodenkathetern äußerst diffizil.

Die US 2006/037195 zeigt einem Elektrodenkatheter gemäß dem Oberbegriff des Anspruchs 1.

Ausgehend von den geschilderten Problemen des Standes der Technik liegt der Erfindung die Aufgabe zugrunde, Elektrodenzuleitungen an eine z.B. Ringelektrode anzubinden und gleichzeitig Zuleitungen für distal dieser Elektrode liegende weitere Elektroden durchzuführen, wobei konstruktionsbedingt geringe isodiametrische Elektrodendurchmesser erreichbar, die Weiterführung von Zuleitungen zu weiter distal gelegenen Elektroden einfacher gestaltet sein sollen und damit insgesamt eine bi- oder multipolare Elektrode mit weniger Aufwand bei der Kontaktierung realisierbar wird.

Diese Aufgabe wird durch die Merkmale des Anspruchs 1 und die im Kennzeichnungsteil des Anspruches 1 angegebene Grundkonzeption gelöst, die charakterisiert ist durch
- eine Steckanschlussverbindung zwischen der ersten Zuleitung und der entsprechenden Elektrode sowie
- eine isolierte Durchführung der zweiten Zuleitung durch diese Elektrode.

In vorteilhafter Weise ergibt sich durch diese Konzeption die Möglichkeit, einen Wechsel zwischen verschiedenen Wendeln proximal und distal einer Elektrode zu realisieren, beispielsweise von einer Vierfach-Wendel proximal vor einer Elektrode auf eine Zweifach-Wendel distal von dieser Elektrode. Bi- und multipolare Elektroden genauso wie unipolare Elektroden können dabei mit identischen Elektrodenformen realisiert werden, was eine Art Baukastensystem ergibt. Gleichzeitig kann eine kompakte Bauweise mit entsprechend kleinem Außendurchmesser des Elektrodenkatheters erreicht werden.

Die abhängigen Ansprüche kennzeichnen vorteilhafte Weiterbildungen der Erfindung, deren Merkmale, Einzelheiten und Vorteile anhand der Beschreibung der Ausführungsbeispiele anhand der beigefügten Zeichnungen näher erläutert werden. Es zeigen:
- Fig. 1: eine Gesamtansicht eines bipolaren Elektrodenkatheters, und
- Fig. 2 bis 4: ausschnittsweise, vergrößerte Längsaxialschnitte des Elektrodenkatheters im Bereich einer Ringelektrode in unterschiedlichen Ausführungsformen.

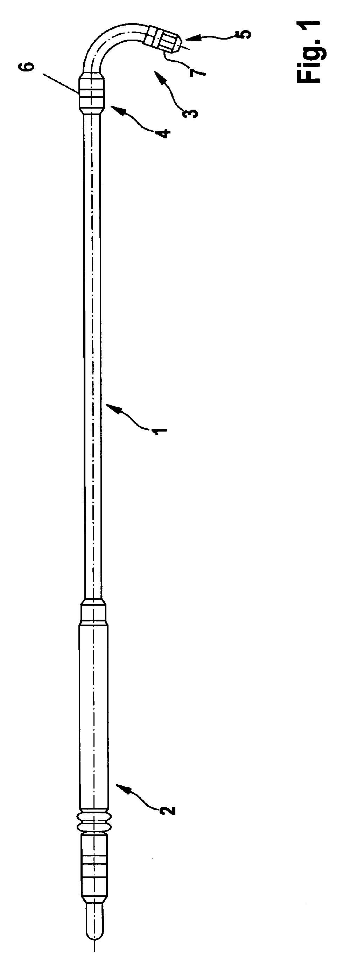

Wie insbesondere aus Figur 1 hervorgeht, weist der zur Kardialtherapie dienende Elektrodenkatheter einen langgestreckten, schlauchartigen Katheterkörper 1 auf, der an seinem proximalen Ende mit einem Anschlussstecker 2 zur Verbindung mit einem entsprechenden Implantat versehen ist. Im Bereich des distalen Endes 3 ist eine Ringelektrode 4 sowie unmittelbar an der Spitze des distalen Endes 3 eine Kopfelektrode 5 angeordnet. Diese Elektroden 4, 5 dienen zur Abgabe oder Messung eines elektrischen Signals, im vorliegenden Falle also eines elektrokardialen Signals über ihre äußere Elektrodenkontaktfläche 6, 7 beispielsweise für eine zuverlässige und effektive Defibrillation oder eine Diagnostik zur Früherkennung von Vorhofflimmern und einer Herzinsuffizienzprogression.

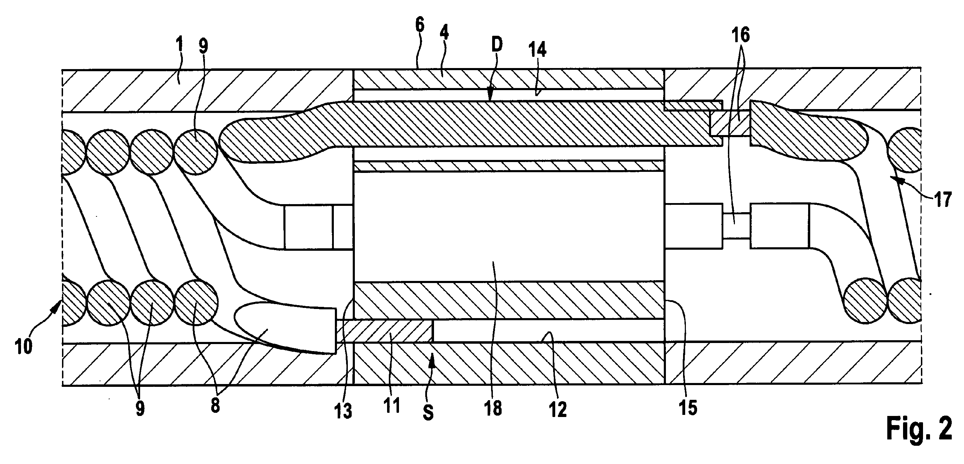

Anhand von Figur 2 ist nun eine erste Ausführungsform für die Anbindung der Elektroden 4, 5 an entsprechende Zuleitungen 8, 9 zu erläutern. So werden in dem schlauchartigen Katheterkörper 1 von proximal her je zwei Zuleitungen 8, 9 in Form einer Vierfach-Wendel 10 herangeführt. Die beiden Zuleitungen 8 für die Ringelektrode 4 sind dabei mit Hilfe einer noch näher zu erläuternden Steckanschlussverbindung S an die Ringelektrode 4 angekoppelt. Die beiden Zuleitungen 9 für die in Figur 2 nicht erkennbare Kopfelektrode 5 (Figur 1) sind mit Hilfe einer als Ganzes mit D bezeichneten Durchführung von proximal nach distal durch die Ringelektrode 4 hindurchgeführt.

Aus der Vierfach-Wendel 10 werden die beiden Zuleitungen 8 in axialer Richtung weggeführt und deren Enden 11 abisoliert. Das jeweilige abisolierte Ende wird in eine axialparallele Aufnahmebohrung 12 oder Aufnahme mit in der innerhalb des Katheterkörpers 1 liegenden, ringförmigen Stirnfläche 13 der Ringelektrode 4 eingesteckt und dort stoffschlüssig, beispielsweise durch Lasern oder Widerstandsschweißen kontaktsicher und mechanisch fest verbunden.

Für die beiden Zuleitungen 9 der Kopfelektrode 5 sind längsaxialparallele Durchführungsbohrungen 14 in der Ringelektrode 4 vorgesehen, die zwischen der proximal liegenden Stirnfläche 13 und der distal liegenden Stirnfläche 15 der Ringelektrode 4 verlaufen. Die axialparallel von der Vierfach-Wendel 10 weggeführten Zuleitungen 9 führen mit diesem isolierten, geraden Schenkel durch die Durchführungsbohrung 14 hindurch. Ihre abisolierten Enden 16 sind an eine Zweifach-Wendel 17 angebunden, die isodiametral bis zur nicht gezeigten Kopfelektrode 5 weiterführt und dort entsprechend angeschlossen ist. Die elektrische Kontaktierung zwischen dem Ende 16 der Zuleitungen 9 und der Zweifach-Wendel 17 kann beispielsweise über eine stoffschlüssige Verbindung hergestellt werden.

Die zentrale Öffnung 18 der Ringelektrode 4 dient ― genauso wie die Innenöffnungen der Wendeln 10, 17 ― für die Durchführung der Mandrins bzw. von Führungsdrähten für den Elektrodenkatheter.

Diese Art der Kontaktierung lässt sich auch auf den Elektrodenkopf und auf den Stecker zu einem Herzschrittmachergerät übertragen. Somit ist der Elektrodenkatheter konstruktionstechnisch als Baukastensystem aufbaubar.

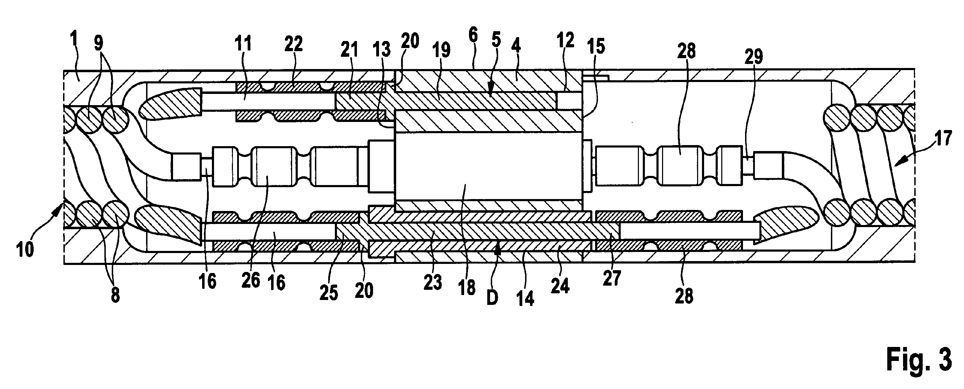

Die in Figur 3 gezeigte Ausführungsform der Steckanschlussverbindung S und der Durchführung D für die Zuleitungen 8, 9 beruht grundsätzlich auf einer Crimptechnik. So weist die Ringelektrode 4 wiederum jeweils zwei zwischen den Stirnflächen 13, 15 längsaxial parallel verlaufende Aufnahme- bzw. Durchführungsbohrungen 12, 14 auf. In den Aufnahmebohrungen 12 sitzt jeweils ein metallischer Crimpschaft 19, der mit der Ringelektrode 4 elektrisch kontaktverbunden und mechanisch beispielsweise durch Einpressen oder stoffschlüssige Verbindung in der Aufnahmebohrung 12 fixiert ist. Die Einstecktiefe des Crimpschaftes 19 ist dabei durch eine Anschlagschulter 20 begrenzt. Außerhalb der Aufnahmebohrung 12 weist der Crimpschaft 19 einen Crimpstift 21 auf. Die abisolierten Enden 11 der Zuleitung 8 für die Ringelektrode 4 sind in eine Crimphülse 22 eingesteckt, die auf dem Crimpstift 21 sitzt. Durch eine übliche Vercrimpung der Crimphülse 22 wird eine sichere mechanische und elektrische Verbindung zwischen den Enden 11 der Zuleitung 8 und dem Crimpschaft 19 geschaffen. Diese Crimpanbindung der Drähte an die Elektrode ist auch als Schweißanbindung auslegbar unter Verwendung gleichartiger Bauteile.

Zur Durchführung der Zuleitung 9 für die in Figur 3 nicht dargestellte Kopfelektrode 5 (Figur 1) ist ein entsprechender Crimpschaft 23 mit einer ihn umgebenden Isolierhülse 24 in die Durchführungsbohrungen 14 in der Ringelektrode 4 eingesetzt. Die Baugruppe aus Crimpschaft 23 und Isolierhülse 24 ist beispielsweise durch Verpressen stabil in der Durchführungsbohrung 14 gehalten. Die Einstecktiefe des Crimpschaftes 23 in die Isolierhülse 24 ist wiederum über eine Anschlagschulter 20 begrenzt, außerhalb derer proximal ein Crimpstift 25 ausgebildet ist. Analog der Anbindung der Zuleitung 8 ist auch hier das abisolierte Ende 16 der Zuleitungen 9 in eine Crimphülse 26 eingesteckt, die auf dem Crimpstift 25 sitzt. Durch eine Vercrimpung dieser Crimphülse 26 wird eine mechanisch und elektrisch stabile Verbindung zwischen den Zuleitungen 9 und den entsprechenden Crimpschäften 23 geschaffen.

Deren distales Ende 27 ragt über die Isolierhülse 24 hinaus und dient zusammen mit einer darauf gesteckten Crimphülse 28 zur Anbindung der abisolierten, axial weggeführten Drähten der distal zur Kopfelektrode 5 führenden Zweifach-Wendel 17.

Auch die Ringelektrode 4 gemäß Figur 3 weist eine zentrale Öffnung 18 zur Durchführung des Mandrins und von Führungsdrähten auf.

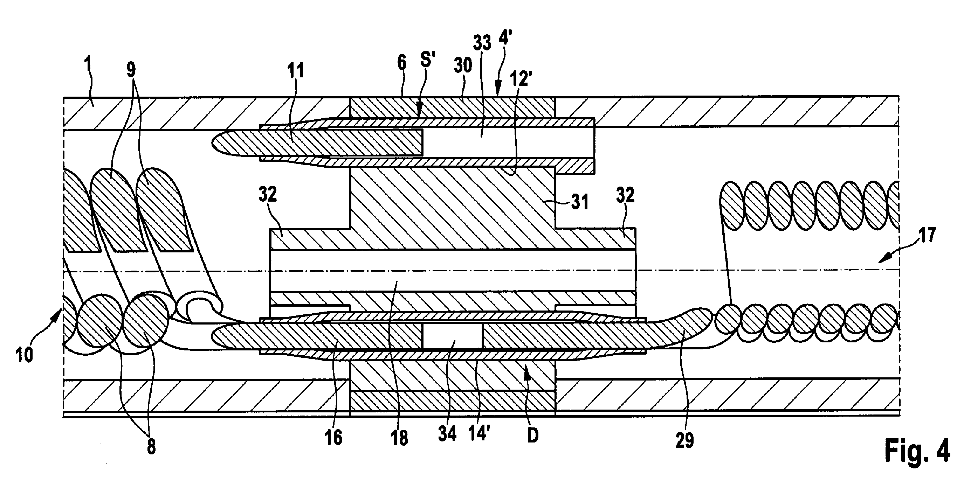

Während die Ringelektrode 4 gemäß den Figuren 2 und 3 als massive Ringelektrode ausgebildet ist, ist bei der in Figur 4 gezeigten Ausführungsform die Ringelektrode 4' mit einem außen liegenden, dünnwandigen Elektrodenring 30 versehen, der die Elektrodenkontaktfläche 6 bildet. In der Ringöffnung sitzt ein im Wesentlichen zylindrischer Kern 31 aus isolierendem Kunststoff, dessen Axiallänge im peripheren Bereich der des Elektrodenrings 30 entspricht. Zentral ist am Kern 31 eine Öffnung 18 vorgesehen, die durch entsprechende Kragenvorsprünge 32 gegenüber der Axiallänge des Kerns 31 verlängert ist. Diese Öffnung 18 dient wiederum zur Durchführung des Mandrins.

Für den Anschluss der beiden Zuleitungen 8 der Ringelektrode 4' sind im Bereich der Peripherie des Kerns 31 axialparallel verlaufende, radial nach außen schlitzartig offene Aufnahmebohrungen 12' vorgesehen, in denen metallische Crimphülsen 33 eingesetzt sind. Diese liegen somit an dem Elektrodenring 30 an und stellen somit eine elektrische Verbindung zu diesem her. Die abisolierten Enden 11 der Zuleitungen 8 stecken in diesen Crimphülsen 33 und sind durch eine entsprechende Vercrimpung mechanisch und elektrisch fest damit verbunden.

Für die Durchführung D der Zuleitungen 9 zur Kopfelektrode 5 hin sind im Kern 31 Durchführungsbohrungen 14' axial parallel eingebracht, die mit Radialabstand von der zentralen Achse und dem Elektrodenring 30 positioniert sind. Damit ist die darin sitzende Crimphülse 34 elektrisch isoliert vom Elektrodenring 30 angeordnet. Die abisolierten Enden 16 der Zuleitungen 9 stecken in den proximalen Enden der Crimphülse 34. In deren distalen Enden sind die abisolierten Enden 29 der Zweifach-Wendel 17 eingesteckt. Durch eine entsprechende Vercrimpung der Crimphülse 34 an ihrem distalen und proximalen Ende ist eine mechanisch und elektrisch sichere Verbindung zwischen der Zweifach-Wendel 17 und den Zuleitungen 9 geschaffen. Diese Crimpanbindung der Drähte an die Elektrode ist auch als Schweißanbindung auslegbar unter Verwendung gleichartiger Bauteile.

Der guten Ordnung halber wird darauf hingewiesen, dass in Figur 4 von den jeweils zwei Steckanschlussverbindungen S und Durchführungen D nur eine einzige in der Schnittdarstellung erkennbar sind. Ferner ist festzuhalten, dass die Steckanschlussverbindungen S und die Durchführungen D gemäß den Figuren 2 und 3 wechselseitig austauschbar sind, dass also eine Steckanschlussverbindung S gemäß Figur 2 mit einer Durchführung D gemäß Figur 3 und umgekehrt kombiniert werden kann.

## Patentansprüche

1. Elektrodenkatheter, insbesondere zur Kardialtherapie, umfassend
- einen langgestreckten, schlauchartigen Katheterkörper (1),
- eine Elektrode (4, 5) zur Abgabe oder Messung eines elektrischen, insbesondere elektrokardialen Signals über ihre äußere Elektrodenkontaktfläche (6, 7),
- mindestens eine erste Zuleitung (8) zum elektrischen Anschluss der Elektrode (4, 4'), und
- mindestens eine zweite Zuleitung (9) zum elektrischen Anschluss einer weiteren Elektrode (5), wobei die erste(n) und zweite(n) Zuleitung(en) (8, 9) gemeinsam als coradiale Mehrfachwendel (10) von proximal zu der Elektrode (4, 4') herangeführt sind,
- eine Steckanschlussverbindung (S) zwischen der ersten Zuleitung (8) und der Elektrode (4, 4'), sowie
- eine isolierte Durchführung (D) der zweiten Zuleitung (9) durch die Elektrode (4, 4'),
**dadurch gekennzeichnet, dass**
- die an die Elektrode (4, 4') anzuschließenden oder durch diese durchzuführenden Enden (11, 16) der Zuleitungen (8, 9) längsaxialparallel aus dem Wendelverbund weggeführt sind,
- die isolierte Durchführung (D) der zweiten Zuleitung(en) (9) in längsaxialparallelen Durchgangsbohrungen (14,14') durch die Elektrode (4, 4') hindurchgeführt sind, und
- die durch die Elektrode (4, 4') durchgeführte(n) zweite(n) Zuleitung(en) (9) mit einer distal weiterführenden Wendelleitung (17) verbunden sind.

2. Elektrodenkatheter nach Anspruch 1, **dadurch gekennzeichnet, dass** die Steckanschlussverbindung (S) als Aufnahmebohrung (12) oder Aufnahmenut in einer im Katheterkörper (1) liegenden Stirnfläche (13) der Elektrode (4, 4') ausgelegt ist, in die das abisolierte Ende (11) der ersten Zuleitung (8) eingesteckt und fixiert ist.

3. Elektrodenkatheter nach Anspruch 1, **dadurch gekennzeichnet, dass** die Steckanschlussverbindung (S) als Crimpverbindung ausgelegt ist, die einen in der Elektrode (4) sitzenden Crimpschaft (19) mit von einer im Katheterkörper (1) liegenden Stirnfläche (13) der Elektrode (4) abstehendem Crimpstift (21) sowie eine Crimpstift (21) und abisoliertes Ende (11) der ersten Zuleitung (8) verbindende Crimphülse (22) aufweist.

4. Elektrodenkatheter nach einem der vorgenannten Ansprüche, **dadurch gekennzeichnet, dass** die Elektrode (4) als massive Ringelektrode ausgebildet ist.

5. Elektrodenkatheter nach Anspruch 1, **dadurch gekennzeichnet, dass** die Elektrode (4') einen außen liegenden, dünnwandigen Elektrodenring (30) und einen isolierenden Kern (31) aufweist, wobei in einer zum Elektrodenring (30) hin offenen Aufnahmebohrung (12') des Kerns (31) eine Crimphülse oder Schweißhülse (33) mit elektrischem Kontakt zum Elektrodenring (30) angeordnet und mit dem abisolierten Ende (11) der ersten Zuleitung (8) vercrimpt oder verschweißt ist.

6. Elektrodenkatheter nach einem der Ansprüche 1 bis 4, **dadurch gekennzeichnet, dass** die isolierte Durchführung (D) für die zweite Zuleitung (9) als Durchgangsbohrung (14) durch die Elektrode (4) ausgebildet ist, durch die die isolierte zweite Zuleitung (9) hindurchgeführt ist.

7. Elektrodenkatheter nach Anspruch 6, **dadurch gekennzeichnet, dass** durchgeführte zweite Zuleitung (9) distal der isolierten Durchführung (D) an eine weiterführende Leitungswendel (17) angeschlossen ist.

8. Elektrodenkatheter nach einem der Ansprüche 1 bis 4, **dadurch gekennzeichnet, dass** die isolierte Durchführung (D) für die zweite Zuleitung (9) durch einen isoliert durch die Elektrode (4) geführten Crimpschaft (23) gebildet ist, der aufweist
- proximal einen von einer im Katheterkörper (1) liegenden Stirnfläche (13) der Elektrode (4) abstehenden Crimpstift (25) sowie eine jeweils Crimpstift (25) und abisoliertes Ende (16) der zweiten Zuleitung (9) verbindende Crimphülse (26), und
- distal einen von einer im Katheterkörper (1) liegenden weiteren Stirnfläche (15) der Elektrode (4) abstehenden Crimpstift (27) sowie eine jeweils diesen Crimpstift (27) und ein abisoliertes Ende (29) einer distal weiterführenden Leitung (17) verbindende Crimphülse (28).

9. Elektrodenkatheter nach Anspruch 3 oder 8, **dadurch gekennzeichnet, dass** der Crimpschaft (19, 23) eine Anschlagschulter (20) zur Begrenzung seiner Einstecktiefe in die Elektrode (4) aufweist.

10. Elektrodenkatheter nach Anspruch 5, **dadurch gekennzeichnet, dass** die zweite Zuleitung (9) durch den isolierenden Kern (31) mit Radialabstand zum Elektrodenring (30) durchgeführt ist.

11. Elektrodenkatheter nach Anspruch 10, **dadurch gekennzeichnet, dass** eine Crimphülse (34) im isolierenden Kern (31) mit Radialabstand zum Elektrodenring (30) durchgeführt ist, die proximal mit dem abisolierten Ende (16) der zweiten Zuleitung (9) und distal mit dem abisolierten Ende (29) einer distal weiterführenden Leitung (17) vercrimpt ist.

12. Elektrodenkatheter nach einem der vorgenannten Ansprüche, **dadurch gekennzeichnet, dass** die erste und zweite Zuleitung (8, 9) mindestens doppelt vorhanden sind.

## Claims

1. An electrode catheter, in particular for cardiac therapy, comprising
- an elongate, tubular catheter body (1),
- an electrode (4, 5) for delivering or measuring an electrical signal, in particular an electrocardiac signal, via its outer electrode contact surface (6, 7),
- at least one first supply line (8) for the electrical connection of the electrode (4, 4'), and
- at least one second supply line (9) for the electrical connection of a further electrode (5),
wherein the first and second supply line(s) (8, 9) are led jointly as a coradial multiple coil (10) from proximally to the electrode (4, 4'),
- a plug terminal connection (S) between the first supply line (8) and the electrode (4, 4'), and
- an insulated passage (D) of the second supply line (9) through the electrode (4, 4'),
**characterised in that**
- the ends (11, 16) of the supply lines (8, 9) to be connected to the electrode (4, 4') or to be passed therethrough are led away from the coil composite in a longitudinally axially parallel manner,
- the insulated passage (D) of the second supply line(s) (9) is passed through the electrode (4, 4') in longitudinally axially parallel through-holes (14, 14'), and
- the second supply line(s) (9) passed through the electrode (4, 4') are connected to a distal coil line (17), which leads on further.

2. The electrode catheter according to Claim 1, **characterised in that** the plug terminal connection (S) is designed as a receptacle hole (12) or receptacle groove in an end face (13), located in the catheter body (1), of the electrode (4, 4'), the stripped end (11) of the first supply line (8) being inserted and fixed in said receptacle hole or groove.

3. The electrode catheter according to Claim 1, **characterised in that** the plug terminal connection (S) is designed as a crimped connection, which has a crimping shaft (19), seated in the electrode (4), having a crimping pin (21) protruding from an end face (13), located in the catheter body (1), of the electrode (4) and a crimping sleeve (22) connecting the crimping pin (21) and stripped end (11) of the first supply line (8).

4. The electrode catheter according to one of the preceding claims, **characterised in that** the electrode (4) is embodied as a solid ring electrode.

5. The electrode catheter according to Claim 1, **characterised in that** the electrode (4') has an outer, thin-walled electrode ring (30) and an insulating core (31), wherein a crimping sleeve or welding sleeve (33) having electrical contact to the electrode ring (30) is situated in a receptacle hole (12') in the core (31), said hole being open toward the electrode ring (30), and is crimped or welded to the stripped end (11) of the first supply line (8).

6. The electrode catheter according to one of Claims 1 to 4, **characterised in that** the insulated passage (D) for the second supply line (9) is embodied as a through-hole (14) through the electrode (4), the insulated second supply line (9) being passed through said through-hole.

7. The electrode catheter according to Claim 6, **characterised in that** the second supply line (9), which is passed through the through-hole, is connected distally from the insulated passage (D) to a conductor coil (17), which leads on further.

8. The electrode catheter according to one of Claims 1 to 4, **characterised in that** the insulated passage (D) for the second supply line (9) is formed by an insulated crimping shaft (23) passed through the electrode (4) and having
- proximally, a crimping pin (25) protruding from an end face (13), in the catheter body (1), of the electrode (4) and a crimping sleeve (26), which connects the crimping pin (25) and stripped end (16) of the second supply line (9), and
- distally, a crimping pin (27) protruding from a further end face (15), in the catheter body (1), of the electrode (4) and a crimping sleeve (28), which connects this crimping pin (27) and a stripped end (29) of a distal line (17), which leads on further.

9. The electrode catheter according to Claim 3 or 8, **characterised in that** the crimping shaft (19, 23) has a stop shoulder (20) for delimiting its insertion depth into the electrode (4).

10. The electrode catheter according to Claim 5, **characterised in that** the second supply line (9) is passed through the insulating core (31) with radial spacing relative to the electrode ring (30).

11. The electrode catheter according to Claim 10, **characterised in that** a crimping sleeve (34) in the insulating core (31) is passed through with radial spacing relative to the electrode ring (30) and is crimped proximally with the stripped end (16) of the second supply line (9) and distally with the stripped end (29) of a distal line (17), which leads on further.

12. The electrode catheter according to one of the preceding claims, **characterised in that** the first and second supply line (8, 9) are provided at least twice.

## Revendications

1. Cathéter à électrodes, destiné en particulier à une thérapie cardiaque, comprenant
- un corps de cathéter (1) oblong du genre tube,
- une électrode (4, 5) pour l'émission ou la mesure d'un signal électrique, en particulier électro-cardiaque, par sa surface de contact extérieure d'électrode (6, 7),
- au moins un premier câble d'alimentation (8) pour le raccordement électrique de l'électrode (4, 4'), et
- au moins un deuxième câble d'alimentation (9) pour le raccordement électrique d'une autre électrode (5),
où les premier(s) et deuxième(s) câbles d'alimentation (8, 9) sont amenés ensemble, en tant que spirale multiple coradiale (10), de manière proximale, vers l'électrode (4, 4'),
- une connexion à fiches (S) entre le premier câble d'alimentation (8) et l'électrode (4, 4'), et
- un passage isolé (D) du deuxième câble d'alimentation (9) à travers l'électrode (4, 4'),
**caractérisé en ce que**
- les extrémités (11, 16) des câbles d'alimentation (8, 9), destinées à être raccordées à l'électrode (4, 4') ou à traverser celle-ci, sont guidées parallèlement dans une direction axiale longitudinale, hors de l'assemblage en spirale,
- le passage isolé (D) du/des deuxième(s) câble(s) d'alimentation (9) traverse des perçages (14, 14') longitudinals, axials et parallèles dans l'électrode (4, 4'), et
- le(s) deuxième(s) câble(s) d'alimentation (9) traversant l'électrode (4, 4') sont reliés à un câble en spirale (17) s'étendant de façon distale.

2. Cathéter à électrodes selon la revendication 1, **caractérisé en ce que** la connexion à fiches (S) est conçue comme un perçage d'admission (12) ou comme une rainure d'admission dans une surface frontale (13) de l'électrode (4, 4') située dans le corps de cathéter (1), dans lequel ou dans laquelle est introduite et fixée l'extrémité isolée (11) du premier câble d'alimentation (8).

3. Cathéter à électrodes selon la revendication 1, **caractérisé en ce que** la connexion à fiches (S) est conçue comme un sertissage comportant un corps à sertir (19) reposant dans l'électrode (4), avec une tige à sertir (21) faisant saillie sur une surface frontale (13) de l'électrode (4) située dans le corps de cathéter (1), ainsi qu'une douille à sertir (22) reliant la tige à sertir (21) et l'extrémité isolée (11) du premier câble d'alimentation (8).

4. Cathéter d'électrode selon l'une des revendications précédentes, **caractérisé en ce que** l'électrode (4) est conçue comme une électrode annulaire massive.

5. Cathéter à électrodes selon la revendication 1, **caractérisé en ce que** l'électrode (4') comporte une bague d'électrode (30) à fine paroi et un noyau isolant (31), dans lequel une douille à sertir ou une douille à souder (33) dotée d'un contact électrique vers la bague d'électrode (30), est disposée dans un perçage d'admission (12') du noyau (31) ouvert vers la bague d'électrode (30), et sertie ou soudée avec l'extrémité isolée (11) du premier câble d'alimentation (8).

6. Cathéter à électrodes selon l'une des revendications 1 à 4, **caractérisé en ce que** le passage isolé (D) pour le deuxième câble d'alimentation (9) est conçu comme un perçage (14) à travers l'électrode (4), dans lequel passe le deuxième câble d'alimentation (9) isolé.

7. Cathéter à électrodes selon la revendication 6, **caractérisé en ce que** le deuxième câble d'alimentation (9) traversant est raccordé à une spirale de câble (17) consécutive, de façon distale par rapport au passage isolé (D).

8. Cathéter à électrodes selon l'une des revendications 1 à 4, **caractérisé en ce que** le passage isolé (D) pour le deuxième câble d'alimentation (9) est formé par un corps à sertir (23) guidé de façon isolée à travers l'électrode (4), comportant
- une tige à sertir (25), faisant saillie sur une surface frontale (13) de l'électrode (4) située dans le corps de cathéter (1), à l'extrémité proximale, ainsi qu'une douille à sertir (26) reliant respectivement la tige à sertir (25) et l'extrémité isolée (16) du deuxième câble d'alimentation (9), et
- une tige à sertir (27) faisant saillie sur une autre surface frontale (15) de l'électrode (4), située dans le corps de corps de cathéter (1), à l'extrémité distale, ainsi qu'une douille à sertir (28) reliant respectivement cette tige à sertir (27) et une extrémité isolée (29) d'un câble (17) s'étendant de façon distale.

9. Cathéter à électrodes selon la revendication 3 ou 8, **caractérisé en ce que** le corps à sertir (19, 23) comporte une épaule de butée (20) pour délimiter sa profondeur d'insertion dans l'électrode (4).

10. Cathéter à électrodes selon la revendication 5, **caractérisé en ce que** le deuxième câble d'alimentation (9) est guidé à travers le noyau isolant (31), avec un espacement radial par rapport à la bague d'électrode (30).

11. Cathéter à électrodes selon la revendication 10, **caractérisé en ce qu'**une douille à sertir (34) est guidée à travers le noyau isolant (31), avec un espacement radial par rapport à la bague d'électrode (30), laquelle est sertie de façon proximale avec l'extrémité isolée (16) du deuxième câble d'alimentation (9) et de façon distale avec l'extrémité isolée (29) d'un câble (17) s'étendant de façon distale.

12. Cathéter à électrodes selon l'une des revendications précédentes, **caractérisé en ce que** le premier et le deuxième câble d'alimentation (8, 9) sont prévus au moins en double.
